# EUROPEAN PATENT SPECIFICATION

(11) **EP 2 104 660 B1**
(45) Date of publication and mention of the grant of the patent: **11.01.2012**
(21) Application number: 07822141.3
(22) Date of filing: 01.11.2007
(51) Int. Cl.: C07C 227/40, C07C 227/18

(54) **A METHOD FOR PREPARING HIGH PURITY 3-CARBOXY-N,N,N-TRIMETHYL-1-PROPANAMINIUM HYDROXIDE INNER SALT**
VERFAHREN ZUR HERSTELLUNG VON HOCHREINEM 3-CARBOXY-N,N,N-TRIMETHYL-1-PROPANAMINIUMHYDROXID (INNERES SALZ)
PROCEDE DE PREPARATION D'UN SEL INTERNE D'HYDROXYDE DE 3-CARBOXY-N,N,N-TRIMETHYL-1-PROPANAMINIUM DE GRANDE PURETE

(30) Priority: 06.11.2006 LV 060123
(43) Date of publication of application: 30.09.2009
(73) Proprietor: LATVIAN INSTITUTE OF ORGANIC SYNTHESIS, 1006 RIGA (LV); Akciju sabiedriba "Olainfarm", Olaine I.V-2114 (LV)
(72) Inventor: Kalvinsh, Ivars, 5052 Ikskile (LV); Chernobrovijs, Aleksandrs, 2114 Olaine (LV); Varacheva, Larisa, 1007 Riga (LV); Pugovichs, Osvalds, 1004 Riga (LV)
(74) Representative: Madgwick, Paul Roland
(86) International application number: PCT/EP2007/061795
(87) International publication number: WO 2008/055843

(56) References cited:
- BREGOFF ET AL: "Paper chromatography of quaternary ammonium bases abd related compounds" J. BIOL. CHEM., vol. 205, 1953, pages 565-574, XP002474190
- Y. CHEVALIER, P. LE PERCHEC: "nterchange distance of flexible zwitterionic molecules in solution." J. PHYS. CHEM., vol. 94, 1990, pages 1768-1774, XP002474191

## Description

The present invention relates to preparing of pharmacologically active compound, namely 3-carboxy-N,N,N-trimethyl-1-propanaminium hydroxide inner salt or gamma-butyrobetaine (hereinafter: GBB), CAS Number 407-64-7.

GBB is known as the precursor of carnitine or vitamin_{BT} biosynthesis in organism (Ch.J.Rebouche, J.Nutr.,113. 1906-1913, 1983), that is used either as dietary supplement for preventing carnitine deficiency (Nutrition Reviews, 36, 10, 308-309, 1978); US 5,030,458), or as pharmacologically active agent for treating carnitine deficiency syndrome (US 4,382,092).

Carnitine deficiency syndrome was described in 1973 by Engel and Angelini (Science 179:899-902), who discovered, that carnitine deficiency in organism is leading to myopathies. Later research demonstrated that GBB also possesses other important pharmacological properties like stimulating antibody production (US 5,569,457), hydroxy-radical scavenging activity (US 5,965,615), etc.

Since preventing carnitine deficiency requires large doses of GBB (up to 20 mg/kg daily), it is important to develop convenient and efficient production methods of this substance with pharmaceutically acceptable purity level (99.5% and higher).

A number of methods for producing GBB are known. They are based either on halogen exchange in gamma-halobutyric acid esters with trimethylamine, or on methylating gamma-aminobutyric or gamma-dimethylaminobutyric acid. The third group of GBB production methods is based on carnitine dehydratation with subsequent hydrogenation (Can.J.Chem.54 (1976), 3310-3311; Synthesis, 1981, 468.)

Unfortunately most of the known GBB syntheses provide for salts of GBB rather than hydrate form. However the production of high purity GBB is essential for development of parenteral formulations, since salts of GBB, especially with ester impurities, are not suitable for preparing parenteral formulations. This is related to toxicity of GBB esters, comparable to that of acetylcholine. Re-crystallization of GBB salts from alcohols is always accompanied by formation of a highly toxic GBB ester. Additionally it should be noted that the purity aspects of GBB salts are not adequately discussed in literature.

A method, proposed by L. Andersom, Th. Kuehler and M. Nilsson (Synthesis, 1981, 46) for preparing GBB hydrochloride to be used in biotechnology applications, was based on methylating N,N-dimethylaminobutyric acid by O-methyl-N,N,-dicyclohexylisourea. Unfortunately the decomposition of the intermediate to GBB is performed by hydrochloric acid, therefore GBB, which is produced in fair yields (68-78%), is isolated only as hydrochloride. A drawback of this method is the price of alkylating agent that should be specially prepared from N,N,-dicyclohexylcarbodiimide. Another drawback is caused by use of 10% excess of the alkylating agent that creates additional problems in industrial application, connected with utilization of waste products. A method for producing gamma-N,N-dimethylaminobutyric acid from N-methylpyrrolidone is not efficient. The total yield from N-methylpyrrolidone in this process is about 41% which is not satisfactory. Still the main problem in using this approach in pharmaceutical production is the fact that purification of GBB as hydrochloride by crystallizing from absolute ethanol is not practically possible due to the formation of small impurity of highly toxic GBB ethyl ester in the crystallization process. Therefore an alternative approach was proposed, based on crystallization of this product from dimethylformamide. However it should be noticed that dimethylformamide is a high boiling solvent and removing it from occlusion in crystals to the level acceptable in parenteral formulations is a difficult and costly process.

A method for producing GBB from gamma-dimethylaminobutyric acid isobutyl ester was described (Plant. Physiol., 1987, 84, 781; Coll.Czech.Chem.Comm., 1930, 2, 712.), starting from gamma-dimethylaminobutyric acid, acetyl chloride and isobutanol. The quaternization of dimethylamino group in this method is performed by methyl iodide in presence of potassium hydrogencarbonate in methanolic solution. Isobutyl trimethylaminiumbutyrate iodide is further extracted by chloroform and hydrolyzed by hydrochloric acid, removing the inorganics by Ag₂O and ionexchange resin. This process is not suitable for industrial use since it is long, costly and inefficient.

Another method for producing GBB from gamma-aminobutyric acid (Zeitschr.f.Biol., 1927, 86, 187) uses methylating by dimethysulfate. It is however not clear how to prepare a high purity GBB hydrate from the salts produced in this process.

Another method for preparing GBB (EP 284292) is based on alkylating two equivalents of trimethylamine by gamma-bromobutyric acid ester in acetone and further hydrolysis of the resulting trimethylaminiumbutanoic acid ethyl ester bromide at pH 12-12.5 by excess potassium hydroxide in aqueous ethanol. The preparation of GBB requires 3-4 days. The reaction product is separated after neutralizing the reaction mixture with 20% sulfuric acid and evaporation.

Unfortunately the purification of the end product is not described and it is not characterized. The product used in further synthesis is described as fairly soluble in chloroform (220.3 grams of product dissolved in 300 grams of chloroform). It should be noticed that hydrates of GBB are practically insoluble in chloroform. Using this method in preparing GBB of pharmaceutical purity level is complicated by another fact, namely the filtering off of KBr from the methanolic BGG solution does not provide for complete removal of KBr.

Aksnes et al. (J.Chem.Soc., 1959, 103) have described preparing of GBB methyl ester in a low 20% yield by boiling methyl gamma-chlorobutyrate with trimethylamine in alcohol. This intermediate may be regarded as the most convenient synthon for producing GBB dihydrate. However the low yield in this process complicates using it in industrial production.

A similar scheme for producing GBB was proposed in US 5,087,745 that is used as prototype for our invention. According to it, trimethylamine is alkylated by gamma-chlorobutyric acid ester in ethanol under pressure in autoclave, ester hydrolyzed by 30% NaOH and most of NaCl separated by filtering off, the rest removed by electrodialysis. The calculated yield in this process (without recovery of product and purification) is 80% and the final product is allegedly obtained in 99.5% purity. However the purification and crystallization is not disclosed and the yield of product is calculated after the total dehydratation of GBB.

The main drawback of this process is that the recovery of GBB in inner salt form (either monohydrate or dihydrate) and purification by electrodialysis can not be performed on standard equipment of chemical facilities, since electrodialysis requires a special equipment and scaling of production requires experimental adjusting of electrodialysis parameters, like current density and flow rate, the membranes are rapidly worn out and pose technical complications in replacing, the electrodes are of special make (platinum coated titanium). Accompanying water electrolysis with production of explosive gases in premises is possible.

Therefore the aim of present invention was to develop a method for producing the inner salt of GBB from salts made via gamma-halobutyric esters, that would be convenient and safe, performed in standard equipment (reactors) of chemical facilities with high yields and high purity of end product and suitable for industrial scale application.

We have surprisingly discovered that treating reaction mixture after basic hydrolysis, namely mixture of 3-carboxy-N,N,N-trimethyl-1-propanaminium hydroxide inner salt and inorganic salts, with carbon dioxide or sulfur dioxide in ethanolic solution, the double salts are destroyed and inorganic salts are completely separated by simple filtration.

It was totally unexpected since all attempts to separate the inorganic salts from GBB by sedimentation with alcohols without treating with CO₂ or SO₂ failed, separating no more than 92% of salts. In conformity with general concept of solubility it was expected that NaCl or KCl that are practically insoluble in alcohol would precipitate when a product, like GBB, containing such salts is dissolved in a solvent where NaCl or KCl are insoluble. However this is not the case with mixture of GBB and inorganic salts like NaCl or KCl, since GBB evidently forms rather stable and soluble double salts with these inorganic salts. Therefore it was still more unexpected that these adducts were destroyed by CO₂ or SO₂ and the inorganic salts become separable from GBB by simple filtering off as disclosed in present invention.

Therefore the present process has substantial advantage to prior art since no electrodialysis or ion exchange resin is necessary in the purification. This aspect makes the scaling simple and the recovery of GBB much easier and effective than in previously known processes, which is a substantial technological advantage of the disclosed process.

As a starting material for this method any easily hydrolyzable ester of 3-trimethylaminiumbutanoic acid as salt is applicable, namely methyl, ethyl, propyl or benzyl ester. As applicable in this method, salts of 3-trimethylaminiumbutanoic acid esters such as halides, methylsulfates, hydrogensulfates, sulfates, phosphates, tosylates and triflates can be used. For the separation of salts such solvents are applicable that do not dissolve the salts produced in the neutralization and basic hydrolysis process, as well as combinations thereof. Especially convenient in use are such solvents as methanol, ethanol, propanol, propanol-2. If necessary such solvents as acetone, methylethylketone or other suitable solvents and combinations thereof can be used.

As the basic agents for saponification such substances as sodium, potassium, lithium, cesium, calcium or magnesium oxides, hydroxides, carbonates, bicarbonates or strong organic bases can be used.

Preparation of gamma-butyrobetaine (GBB) according to one version of the present invention is shown in the following reaction scheme:

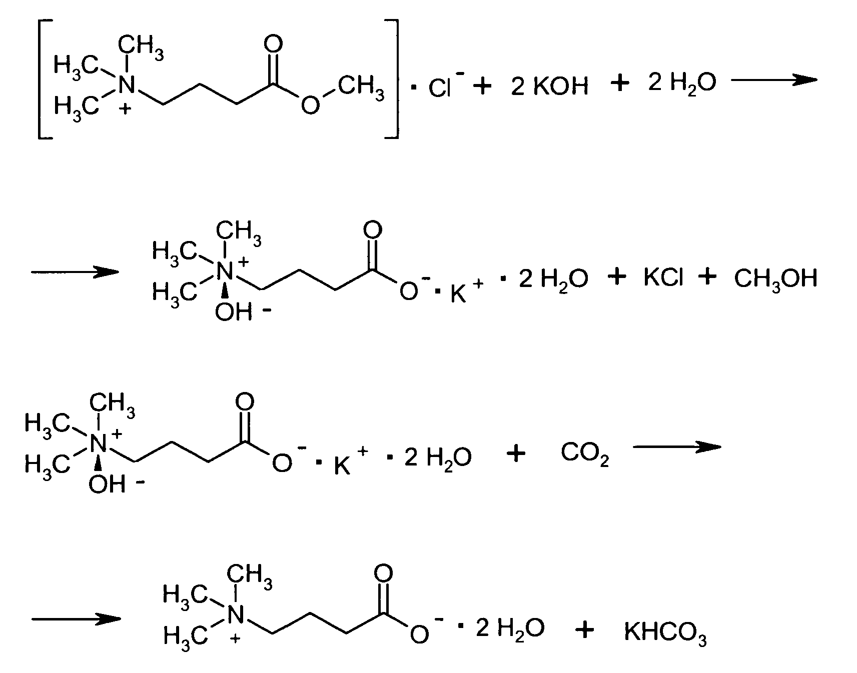

The disclosed process is illustrated but not limited by the following examples.

### Example 1

Potassium hydroxide (20 kg) was dissolved in 96% ethanol (170 l) and cooled to 20±2 °C. 3-trimethylaminiumbutanoic acid methyl ester chloride (30 kg) was added and vigorously stirred for 1 - 3 h at 18 - 23 °C till complete saponification (chromatographic control). Reaction mixture was cooled to 2 - 5 °C, held for 30 - 60 min and the precipitate of KCI filtered of. The precipitate (KCl, 10,7 kg, ∼92%) was washed with ethanol (3 x 20 l). Filtrates were pooled and at 20±5 °C saturated with carbon dioxide to pH ∼8 - 8.5 (by pH-meter) under vigorous stirring and cooling.

Reaction mass was cooled to 15 - 18 °C and the precipitate filtered off. The precipitate was washed by suspending in ethanol (4 x 20 l). The filtrates were pooled and evaporated in vacuo at 45 - 50 °C. The residue was dissolved in absolute propanol-2 (50 l) and evaporated in vacuo. This procedure was repeated until the water content in the removed propanol-2 was below 2%. The dry residue was dissolved in absolute propanol-2 (300 l), kept at 1 - 3 °C for 10 - 12 h and the precipitate of inorganic salts (∼ 250 g) filtered off. Filtrate was concentrated in vacuo at 45 - 50 °C, removing about 280 l of propanol-2. Acetone (100 l) was added with stirring. The reaction mass was cooled to 25 °C and kept for ∼3 - 5 h. The precipitate was filtered off, washed with dry acetone (20 l) and dried in vacuo at 35-40 °C. 3-carboxy-N,N,N-trimethyl-1-propanaminium hydroxide inner salt (19.4-21.0kg) with purity 99.5% and higher was obtained. The amount of crystallization water (1 or 2 molecules) in the crystalline product is dependant on the conditions of drying. If necessary the product can be re-crystallized from propanol-2.

### Example 2

Potassium hydroxide (20 kg) was dissolved in 96% ethanol (170 l) and cooled to 20±2 °C. 3-trimethylaminiumbutanoic acid methyl ester chloride (30 kg) was added and vigorously stirred for at 18 - 23 °C till complete saponification (chromatographic control). Reaction mixture was cooled to 2 - 5° C, held for 30 min and the precipitate of KCl filtered of. The precipitate (KCl, 10,7 kg, ∼92%) was washed with ethanol (3 x 20 l). Filtrates were pooled and at 20±5 °C saturated with sulfur dioxide to pH ∼8 - 8.5 (by pH-meter) under vigorous stirring and cooling.

Reaction mass was cooled to 15 - 18 °C, the precipitate filtered off and washed by suspending in ethanol (4 x 20 l). The filtrates were pooled and evaporated in vacuo at 45 - 50 °C. The residue was dissolved in absolute propanol-2 (50 l) and concentrated in vacuo. This procedure was repeated until the water content in the removed propanol-2 was below 2%. The dry residue was dissolved in absolute propanol-2 (300 l) and kept at 1 - 3 °C for 10 - 12 h. The precipitate of inorganic salts (∼ 420 g) was filtered off and filtrate concentrated in vacuo at 45 - 50 °C, removing about 280 l of propanol-2. Acetone (100 l) was added to the residue with stirring. The reaction mass was cooled to 2 - 5 °C and kept for ∼3 - 5 h. The precipitate was filtered off, washed with dry acetone (20 l) and dried in vacuo at 35 - 40 °C. 3-carboxy-N,N,N-trimethyl-1-propanaminium hydroxide inner salt (19.0 - 20.5 kg) with purity 99.5% and higher was obtained. The amount of crystallization water (1 or 2 molecules) in the crystalline product is dependant on the conditions of drying. If necessary the product can be re-crystallized from propanol-2.

### Example 3

Potassium hydroxide (20 kg) was dissolved in 96% ethanol (170 l) and cooled to 20±2 °C. 3-trimethylaminiumbutanoic acid methyl ester chloride (30 kg) was added and vigorously stirred for at 18 - 23 °C till complete saponification (chromatographic control). The reaction mixture was cooled to 2 - 5 °C, kept for 30 min and at 20±5 °C saturated with carbon dioxide under vigorous stirring and cooling.

Reaction mass was cooled to 15 - 18 °C and the precipitate filtered off. The precipitate was washed by suspending in ethanol (4 x 30 l). The filtrates were pooled and evaporated in vacuo at 45 - 50 °C. The residue was dissolved in absolute propanol-2 (50 l) and concentrated in vacuo. This procedure was repeated until the water content in the removed propanol-2 was below 2%. The dry residue was dissolved in absolute propanol-2 (300 l), kept at 1 - 3 °C for 10 - 12 h and the precipitate of inorganic salts (∼ 230 g) filtered off. The filtrate was concentrated in rotary evaporator at 45 - 50 °C in the water bath, removing about 280 l of propanol-2, and acetone (100 l) added. The reaction mass was cooled to 2 - 5 °C and kept for ∼3 - 5 h. The precipitate was filtered off, washed with dry acetone (20 l) and dried in vacuo at 35 - 40 °C. 3-carboxy-N,N,N-trimethyl-1-propanaminium hydroxide inner salt (19.7 - 20.8 kg) with purity 99.5% and higher was obtained. The amount of crystallization water (1 or 2 molecules) in the crystalline product is dependant on the conditions of drying. If necessary the product can be re-crystallized from propanol-2.

### Example 4

Potassium hydroxide (20 kg) was dissolved in 96% ethanol (170 l) and cooled to 20±2 °C. 3-trimethylaminiumbutanoic acid methyl ester chloride (30 kg) was added and vigorously stirred for at 18 - 23 °C till complete saponification (chromatographic control). Reaction mixture was cooled to 2 - 5 °C and at 20±5 °C saturated with sulfur dioxide to pH ∼8 - 8.5 (by pH-meter) under vigorous stirring and cooling.

Reaction mass was cooled to 15 - 18 °C, the precipitate filtered off and washed by suspending in ethanol (4 x 30 l). The filtrates were pooled and evaporated in vacuo at 45 - 50 °C. The residue was dissolved in absolute propanol-2 (50 l) and concentrated in vacuo. This procedure was repeated until the water content in the removed propanol-2 was below 2%. The dry residue was dissolved in absolute propanol-2 (300 l), kept at 1 - 3 °C for 10 -12 h. The precipitate of inorganic salts was filtered off and filtrate concentrated in vacuo at 45 - 50 °C, removing about 280 l of propanol-2. The reaction mass was cooled to 2 - 5 °C and kept for ∼3 - 5 h. The precipitate was filtered off, washed with dry acetone (20 l) and dried in vacuo at 35 - 40 °C. 3-carboxy-N,N,N-trimethyl-1-propanaminium hydroxide inner salt (19.3 - 20.9 kg) with purity 99.5% and higher was obtained. The amount of crystallization water (1 or 2 molecules) in the crystalline product is dependant on the conditions of drying. If necessary the product can be re-crystallized from propanol-2.

### Example 5

Sodium hydroxide (14.258 kg) was dissolved in 96% ethanol and cooled to 20±2 °C. 3-trimethylaminiumbutanoic acid methyl ester chloride (30 kg) was added and vigorously stirred for 1 - 3 h at 18 - 23 °C till complete saponification (chromatographic control). Reaction mixture was cooled to 2 - 5 °C, held for 30 - 60 min and the precipitate of NaCl filtered of. The precipitate was washed by ethanol (3 x 20 l). Filtrates were pooled and at 20±5 °C saturated with carbon dioxide to pH ∼8 - 8.5 (by pH-meter) under vigorous stirring and cooling.

Reaction mass was cooled to 15 - 18 °C, the precipitate filtered off and washed by suspending in ethanol (4 x 20 l). The filtrates were pooled and evaporated in vacuo at 45 - 50 °C. The residue was dissolved in absolute propanol-2 (50 l) and concentrated in vacuo. This procedure was repeated until the water content in the removed propanol-2 was below 2%. The dry residue was dissolved in absolute propanol-2 (300 l), kept at 1 - 3 °C for 10 - 12 h and the precipitate of inorganic salts filtered off. The filtrate was concentrated in vacuo at 45 - 50 °C, removing about 280 l of propanol-2, and acetone (100 l) added with stirring. The reaction mass was cooled to 2 - 5 °C and kept for ∼3 - 5 h. The precipitate was filtered off, washed with dry acetone (20 l) and dried in vacuo at 35 - 40 °C. 3-carboxy-N,N,N-trimethyl-1-propanaminium hydroxide inner salt (19.1 - 20.0 kg) with purity 99.5% and higher was obtained. The amount of crystallization water (1 or 2 molecules) in the crystalline product is dependant on the conditions of drying. If necessary the product can be re-crystallized from propanol-2.

### Example 6

Potassium hydroxide (20 kg) was dissolved in 96% ethanol (170 l) and cooled to 20±2 °C. 3-trimethylaminiumbutanoic acid methyl ester chloride (30 kg) was added and vigorously stirred at 18 - 23 °C till complete saponification (chromatographic control). Reaction mixture was cooled to 2 - 5 °C, held for 30 min and the precipitate of KCl filtered of. The precipitate (KCl, 10,7 kg, -92%) was washed with ethanol (3 x 20 l). Filtrates were pooled and at 20±5 °C saturated with sulfur dioxide to pH -8 - 8.5 (by pH-meter) under vigorous stirring and cooling.

Reaction mass was cooled to 15 - 18 °C, the precipitate filtered off and washed by suspending in ethanol (4 x 20 l). The filtrates were pooled and evaporated in vacuo at 45 - 50 °C. The residue was dissolved in absolute propanol-2 (50 l) and concentrated in vacuo. This procedure was repeated until the water content in the removed propanol-2 was below 2%. The dry residue was dissolved in absolute propanol-2 (300 l), kept at 1 - 3 °C for 10 - 12 h and the precipitate of inorganic salts filtered off. The filtrate was concentrated in vacuo at 45 - 50 °C, removing about 280 l of propanol-2, and acetone (100 l) added with stirring. The reaction mass was cooled to 2 - 5 °C and kept for ∼3 - 5 h. The precipitate was filtered off, washed with dry acetone (20 l) and dried in vacuo at 35 - 40 °C. 3-carboxy-N,N,N-trimethyl-1-propanaminium hydroxide inner salt (19.4 - 21.0 kg) with purity 99.5% and higher was obtained. The amount of crystallization water (1 or 2 molecules) in the crystalline product is dependant on the conditions of drying. If necessary the product can be re-crystallized from propanol-2.

### Example 7

Potassium hydroxide (49.264 kg) was dissolved in 96% ethanol (170 l) and cooled to 20±2 °C. 3-trimethylaminiumbutanoic acid methyl ester chloride (30 kg) was added and vigorously stirred at 18 - 23 °C till complete saponification (chromatographic control). Reaction mixture was cooled to 2 - 5 °C, held for 30 - 60 min, the inorganic precipitate filtered of and washed with ethanol (3 x 20 l). Filtrates were pooled and at 20±5 °C saturated with carbon dioxide to pH ∼8 - 8.5 (by pH-meter) under vigorous stirring and cooling.

Reaction mass was cooled to 15 - 18 °C, the precipitate filtered off and washed by suspending in ethanol (4 x 20 l). The filtrates were pooled and evaporated in vacuo at 45 - 50 °C. The residue was dissolved in absolute propanol-2 (50 l) and concentrated in vacuo. This procedure was repeated until the water content in the removed propanol-2 was below 2%. The dry residue was dissolved in absolute propanol-2 (300 ), kept at 1 - 3 °C for 10 - 12 h and the precipitate of inorganic salts filtered off. Filtrate was concentrated in vacuo, removing about 280 l of propanol-2. The residue was treated with acetone (100 l), cooled to 2 - 5 °C and kept for ∼3 - 5 h. The precipitate was filtered off, washed with dry acetone (20 l) and dried in vacuo at 35-40 °C. 3-carboxy-N,N,N-trimethyl-1-propanaminium hydroxide inner salt (19.6 - 21.3 kg) with purity 99.5% and higher was obtained. The amount of crystallization water (1 or 2 molecules) in the crystalline product is dependant on the conditions of drying. If necessary the product can be re-crystallized from propanol-2.

### Example 8

Potassium hydroxide (10 kg) was dissolved in 96% ethanol (170 l) and cooled to 20±2 °C. 3-trimethylaminiumbutanoic acid methyl ester chloride (30 kg) was added and vigorously stirred for 1 - 3 h at 18 - 23 °C till complete saponification (chromatographic control). Reaction mixture was cooled to 2 - 5 °C, held for 30 min and filtered. The precipitate was washed with ethanol (3 x 20 l), filtrates were pooled and at 20±5 °C saturated with carbon dioxide to pH ∼8-8.5 (by pH-meter) under vigorous stirring and cooling.

Reaction mass was cooled to 15 -18 °C, the precipitate filtered off and washed by suspending in ethanol (4 x 20 l). The filtrates were pooled and evaporated in vacuo at 45 - 50 °C. The residue was dissolved in absolute propanol-2 (50 l) and concentrated in vacuo. This procedure was repeated until the water content in the removed propanol-2 was below 2%. The dry residue was dissolved in absolute propanol-2 (300 l), kept at 1 - 3 °C for 10 - 12 h. The precipitate of inorganic salts was filtered off and filtrate concentrated in vacuo at 45 - 50 °C, removing about 280 l of propanol-2. The residue was treated with acetone (100 l), the reaction mass was cooled to 25 °C and kept for -3 - 5 h. The precipitate was filtered off, washed with dry acetone (20 l) and dried in vacuo at 35 - 40 °C. 3-carboxy-N,N,N-trimethyl-1-propanaminium hydroxide inner salt (19.5-21.05 kg) with purity 99.5% and higher was obtained. The amount of crystallization water (1 or 2 molecules) in the crystalline product is dependant on the conditions of drying. If necessary the product can be re-crystallized from propanol-2.

## Claims

1. A method for preparing 3-carboxy-N,N,N-trimethyl-l-propanaminium hydroxide inner salt from 3-trimethylaminiumbutanoic acid ester salt by basic hydrolysis, **characterized in that** the final product is purified from inorganic compounds by saturating its solution in an alcohol by a gaseous acid anhydride and the inorganic salts separated by known methods of precipitate removal.

2. The method of claim 1, **characterized in that** the 3-trimethylaminiumbutanoic acid ester salt is a halide salt.

3. The method of claim 1, **characterized in that** the 3-trimethylaminiumbutanoic acid ester salt is a methylsulfate salt.

4. The method of claim 1, **characterized in that** the 3-trimethylaminiumbutanoic acid ester salt is a tosylate salt.

5. The method of claim 1, **characterized in that** the 3-trimethylaminiumbutanoic acid ester salt is a methyl ester salt.

6. The method of claim 1, **characterized in that** the 3-trimethylaminiumbutanoic acid ester salt is an ethyl ester salt.

7. The method of claim 1, **characterized in that** the 3-trimethylaminiumbutanoic acid ester salt is a benzyl ester salt.

8. The method of claim 1, **characterized in that** the basic hydrolysis agent is selected from a group, containing sodium, potassium, lithium, cesium calcium and magnesium oxides, hydroxides, carbonates, bicarbonates and mixtures thereof.

9. The method of claim 1, **characterized in that** the basic agent is used in molar ratio between 1.05 and 3.15 to said ester salt.

10. The method of claim 1, **characterized in that** the basic agent is potassium hydroxide.

11. The method of claim 1, **characterized in that** the basic agent is sodium hydroxide.

12. The method of claim 1, **characterized in that** the basic agent is a mixture of potassium hydroxide and potassium carbonate.

13. The method of claim 1, **characterized by** that the alcohol is selected from a group, consisting of methanol, ethanol and propanol-2.

14. The method of claim 1, **characterized in that** the acid anhydride is carbon dioxide.

15. The method of claim 1, **characterized in that** the acid anhydride is sulfur dioxide.

## Patentansprüche

1. Ein Verfahren zur Herstellung von zwitterionischem 3-Carboxy-N,N,N,-trimethyl-1-propanaminiumhydroxid-Salz aus einem 3-Trimethylaminiumbutansäureester-Salz durch basische Hydrolyse, **dadurch gekennzeichnet, dass** das Endprodukt von anorganischen Verbindungen gereinigt wird, indem seine Lösung in einem Alkohol durch ein gasförmiges Säureanhydrid gesättigt wird und die anorganischen Salze durch bekannte Fällungsverfahren abgetrennt werden.

2. Das Verfahren nach Anspruch 1, **dadurch gekennzeichnet, dass** das 3-Trimethylaminiumbutansäureester-Salz ein Halogenidsalz ist.

3. Das Verfahren nach Anspruch 1, **dadurch gekennzeichnet, dass** das 3-Trimethylaminiumbutansäureester-Salz ein Methylsulfatsalz ist.

4. Das Verfahren nach Anspruch 1, **dadurch gekennzeichnet, dass** das 3-Trimethylaminiumbutansäureester-Salz ein Tosylatsalz ist.

5. Das Verfahren nach Anspruch 1, **dadurch gekennzeichnet, dass** das 3-Trimethylaminiumbutansäureester-Salz ein Methylestersalz ist.

6. Das Verfahren nach Anspruch 1, **dadurch gekennzeichnet, dass** das 3-Trimethylaminiumbutansäureester-Salz ein Ethylestersalz ist.

7. Das Verfahren nach Anspruch 1, **dadurch gekennzeichnet, dass** das 3-Trimethylaminiumbutansäureester-Salz ein Benzylestersalz ist.

8. Das Verfahren nach Anspruch 1, **dadurch gekennzeichnet, dass** das basische Hydrolysemittel aus einer Gruppe enthaltend Natrium-, Kalium-, Lithium-, Cäsium-, Calcium- und Magnesiumoxide, -hydroxide, -carbonate, -hydrogencarbonate und deren Gemische, ausgewählt wird

9. Das Verfahren nach Anspruch 1, **dadurch gekennzeichnet, dass** das basische Mittel in einem molaren Verhältnis zu dem Estersalz zwischen 1,05 und 3,15 verwendet wird.

10. Das Verfahren nach Anspruch 1, **dadurch gekennzeichnet, dass** das basische Mittel Kaliumhydroxid ist.

11. Das Verfahren nach Anspruch 1, **dadurch gekennzeichnet, dass** das basische Mittel Natriumhydroxid ist.

12. Das Verfahren nach Anspruch 1, **dadurch gekennzeichnet, dass** das basische Mittel ein Gemisch von Kaliumhydroxid und Kaliumcarbonat ist.

13. Das Verfahren nach Anspruch 1, **dadurch gekennzeichnet, dass** der Alkohol aus einer Gruppe bestehend aus Methanol, Ethanol und 2-Propanol ausgewählt wird.

14. Das Verfahren nach Anspruch 1, **dadurch gekennzeichnet, dass** das Säureanhydrid Kohlendioxid ist.

15. Das Verfahren nach Anspruch 1, **dadurch gekennzeichnet, dass** das Säureanhydrid Schwefeldioxid ist.

## Revendications

1. Procédé de préparation d'un sel interne d'hydroxyde de 3-carboxy-N,N,N-triméthyl-1-propanaminium à partir d'un sel d'ester d'acide 3-triméthylaminium butanoïque par hydrolyse basique, **caractérisé en ce que** le produit final est purifié à partir de composés inorganiques en saturant sa solution dans un alcool par un anhydride d'acide gazeux et les sels inorganiques séparés par des procédés connus d'élimination du précipité.

2. Procédé selon la revendication 1, **caractérisé en ce que** le sel d'ester d'acide 3-triméthylaminium butanoïque est un sel d'halogénure.

3. Procédé selon la revendication 1, **caractérisé en ce que** le sel d'ester d'acide 3-triméthylaminium butanoïque est un sel de méthylsulfate.

4. Procédé selon la revendication 1, **caractérisé en ce que** le sel d'ester d'acide 3-triméthylaminium butanoïque est un sel de tosylate.

5. Procédé selon la revendication 1, **caractérisé en ce que** le sel d'ester d'acide 3-triméthylaminium butanoïque est un sel d'ester méthylique.

6. Procédé selon la revendication 1, **caractérisé en ce que** le sel d'ester d'acide 3-triméthylaminium butanoïque est un sel d'ester éthylique.

7. Procédé selon la revendication 1, **caractérisé en ce que** le sel d'ester d'acide 3-triméthylaminium butanoïque est un sel d'ester benzylique.

8. Procédé selon la revendication 1, **caractérisé en ce que** l'agent d'hydrolyse basique est choisi parmi un groupe comprenant des oxydes, hydroxydes, carbonates et bicarbonates de sodium, de potassium, de lithium, de césium et de calcium et des mélanges de ceux-ci.

9. Procédé selon la revendication 1, **caractérisé en ce que** l'agent basique est utilisé en un rapport molaire de 1,05 à 3,15 par rapport au sel d'ester.

10. Procédé selon la revendication 1, **caractérisé en ce que** l'agent basique est l'hydroxyde de potassium.

11. Procédé selon la revendication 1, **caractérisé en ce que** l'agent basique est l'hydroxyde de sodium.

12. Procédé selon la revendication 1, **caractérisé en ce que** l'agent basique est un mélange d'hydroxyde de potassium et de carbonate de potassium.

13. Procédé selon la revendication 1, **caractérisé en ce que** l'alcool est choisi parmi un groupe comprenant le méthanol, l'éthanol et le propanol-2.

14. Procédé selon la revendication 1, **caractérisé en ce que** l'anhydride d'acide est le dioxyde de carbone.

15. Procédé selon la revendication 1, **caractérisé en ce que** l'anhydride d'acide est le dioxyde de soufre.
